# EUROPEAN PATENT APPLICATION

(11) **EP 4 703 006 A1**
(43) Date of publication of application: **04.03.2026**
(21) Application number: 24196667.0
(22) Date of filing: 27.08.2024
(51) Int. Cl.: A61N 5/06

(54) **DEVICE FOR EMITTING LIGHT WITHIN AND OUTSIDE A BODY CAVITY**

(71) Applicant: Uvisa Health ApS, 2300 København S (DK)
(72) Inventor: Harris, Ella, 2300 Copenhagen S (DK); Pendharkar Kulkarni, Sonal, 2730 Herlev (DK); Thams, Christian, 2200 Copenhagen N (DK)
(74) Representative: Nordic Patent Service A/S

(57) **Abstract**

A device (1) for emitting light within and outside a body cavity, the device comprising a housing (2) comprising an intracorporeal arm (3) having a first center axis (A1) and an extracorporeal arm (4) having a second center axis (A2), said first center axis (A1) intersecting said second center axis (A2). The housing (2) comprises a translucent material configured to allow propagation of light through said housing (2). A sensor arrangement (7) is configured to detect if said intracorporeal arm (3) is inserted within said body cavity. A microprocessor (8) is configured to allow activation of at least one light source (5) arranged within said housing (2) and to allow continuous emission of light as long as said sensor arrangement detects that said intracorporeal arm is inserted within said body cavity. A user interface (9) is connected to said microprocessor (8) for activating said light source (s) (5).

## Description

### TECHNICAL FIELD

The disclosure relates to a device for emitting light within, and on tissues arranged outside, a body cavity, the device comprising a housing, an energy source, and a plurality of light sources.

### BACKGROUND

Vaginitis is a condition commonly characterized by pain, itching, discomfort, or unusual discharge, and it is most commonly caused by fungal and bacterial infections. Many women experience this condition recurrently throughout their lives. The condition can arise for several different reasons but is usually a result of a change in the composition of the vaginal microbiome. This can occur due to, for example, hormonal changes, the introduction of foreign pathogens, sensitivity to external compounds often found in personal care products such as soaps, detergents, or tampons, or the introduction of semen.

A typical, known type of treatment comprises the application of antibiotics for bacterial vaginosis, or antifungal medication for fungal infections, most often vulvovaginal Candidiasis. Antibiotics are systemic and may cause side effects such as nausea, vomiting, diarrhea, and headaches. Furthermore, antibiotics dominantly target bacteria, including both commensal and pathogenic species, leaving the vaginal microbiome susceptible to colonization by fungal species, in turn leading to fungal infections. It has also been documented that Gardnerella vaginalis, the bacteria most commonly associated with bacterial vaginosis, has shown a propensity for quickly developing antibiotic resistance when treated repeatedly with antibiotics. A similar tendency is observed in fungal species becoming resistant to antifungal treatments.

An alternative treatment comprises intracorporeal light therapy, for example by means of a UV-emitting device inserted into the vagina. One disadvantage of using such a device is that UV light is damaging to the eye's surface tissues as well as the cornea and lens. A further challenge is associated with consistently applying a very precisely specified UV light dosage. Additionally, the device must be safe and easy to insert and use, in order to ensure this type of therapy constitutes an improvement over other types of care, and that treatment is delivered fully and correctly.

Hence, a safer and more efficient device for emitting light within a body cavity, and on tissue outside said body cavity, such as the vagina and vulva, would be advantageous.

### SUMMARY

It is an object of the invention to provide an improved device for emitting light within a body cavity as well as on tissues surrounding the body cavity, which device is easy to operate and safe for the user.

According to a first aspect, there is provided a device for emitting light within a body cavity and/or on tissue outside the body cavity, the device comprising a housing comprising an intracorporeal arm having a first center axis and an extracorporeal arm having a second center axis, the first center axis intersecting the second center axis, the housing comprising a translucent material configured to allow propagation of light through the housing. A plurality of light sources and an energy source are arranged within the housing. A sensor arrangement is configured to detect if the intracorporeal arm is inserted, to a predefined extent, within the body cavity. A microprocessor is configured to allow activation of at least one of the light sources and to allow continuous emission of light from the light source(s) as long as the sensor arrangement detects that the intracorporeal arm is inserted, to the predefined extent, within the body cavity. A user interface is connected to the microprocessor for activating the light source(s) .

This device provides an option for simple, safe, and efficient at-home treatment of fungal and bacterial infections, and the device does not require special training or protective equipment. The light sources facilitate a decrease in unwanted pathogens and/or microorganisms within and outside of the body cavity, while eliminating the risk of eye-damaging light wavelengths being emitted outside of the body cavity.

In a possible implementation form of the first aspect, the intracorporeal arm is located, to a predefined extent, within the body cavity when all light sources of the intracorporeal arm are located within the body cavity. This prevents light of eye-damaging wavelengths from being emitted outside of the body cavity.

In a further possible implementation form of the first aspect, the sensor arrangement is configured to detect if the intracorporeal arm is located, to a predefined extent, within the body cavity by detecting that the intracorporeal arm has been inserted into an orifice enveloping the intracorporeal arm after insertion. This ensures all light sources, capable of emitting light of eye-damaging wavelengths, have to be located within the body cavity in order to emit light.

In a further possible implementation form of the first aspect, the device is used for vaginal light therapy, facilitating simple and efficient home treatment of for example vaginitis.

In a further possible implementation form of the first aspect, the energy source is a battery and the battery is arranged within the extracorporeal arm. This allows the device to be used without a power cable and facilitates a reduced intracorporeal arm form factor. Furthermore, device safety is improved by ensuring the battery remains outside of the body during use.

In a further possible implementation form of the first aspect, the housing is substantially L-, V- or T-shaped, the L-, V- or T-shape being formed by the intracorporeal arm and the extracorporeal arm. This facilitates simple maneuvering upon insertion and extraction, as well as allows treatment both internally within the body cavity and of tissue outside of the body cavity.

In a further possible implementation form of the first aspect, the first center axis extends at an angle to the second center axis, the angle being 45°<α≤90°, preferably 75°≤α≤90°. This facilitates simple maneuvering upon insertion and extraction, as well as allows treatment both internally within the body cavity as well as of tissue outside of the body cavity.

In a further possible implementation form of the first aspect, the intracorporeal arm and at least a part of the extracorporeal arm is a single-piece unit, ensuring a completely fluid-proof barrier between the body cavity and the interior of the device.

In a further possible implementation form of the first aspect, each light source is deactivated by means of the user interface, by means of a timer connected to the microprocessor, and/or by means of the sensor arrangement detecting extraction of the intracorporeal arm from the body cavity. This allows flexibility regarding how to deactivate the light sources and, hence, adaptability to specific use cases.

In a further possible implementation form of the first aspect, the user interface is an analogue interface arranged on the extracorporeal arm and/or a digital interface downloaded on a digital apparatus connected to the device. This allows device operation to be initiated either directly on the device or remotely.

In a further possible implementation form of the first aspect, the analogue interface is a button and the digital interface is an application. This allows device operation to be initiated either on the device or via, e.g., a smartphone.

In a further possible implementation form of the first aspect, the device further comprises wireless technology allowing transmission of data from the microprocessor to the user interface and, optionally, from the user interface to the microprocessor. This facilitates one-way or two-way communication between the device and the user.

In a further possible implementation form of the first aspect, the sensor arrangement comprises a plurality of sensors arranged in the intracorporeal arm, ensuring enclosure of the intracorporeal arm by the body cavity can be detected both lengthwise and circumferentially.

In a further possible implementation form of the first aspect, the sensor arrangement is configured to sequentially detect the insertion of the intracorporeal arm into the body cavity and/or extraction of the intracorporeal arm from the body cavity, the sequential detection being achieved by means of at least one array of sensors arranged in the intracorporeal arm, the array extending substantially parallel with the first center axis. This allows detecting whether the intracorporeal arm has been sufficiently inserted into the body cavity such that no harmful light can be emitted outside of the body cavity. This, in turn, ensures no light sources are activated erroneously due to, e.g., holding by enclosing the intracorporeal arm with the palm of the hand.

In a further possible implementation form of the first aspect, the sensor arrangement comprises at least one of a capacitive sensor, a light sensor, and/or a temperature sensor, allowing full flexibility and increased optional functionality and usability.

In a further possible implementation form of the first aspect, the plurality of light sources are arranged in the intracorporeal arm and/or in the extracorporeal arm, facilitating light emission internally within the body cavity, externally outside the body cavity, or both.

In a further possible implementation form of the first aspect, the plurality of light sources comprises light-emitting diodes (LEDs) configured to emit light in a 380-500 nm spectrum and, optionally, light-emitting diodes (LEDs) configured to emit light in a 315-400 nm spectrum. This allows use of visible spectrum LEDs and, optionally, UV-A LEDs.

In a further possible implementation form of the first aspect, the light sources configured to emit light in the 380-500 nm spectrum are arranged in the intracorporeal arm and in the extracorporeal arm, while the light sources configured to emit light in the 315-400 nm spectrum are arranged in the intracorporeal arm only. This ensures non-eye-damaging light can be radiated both inside and outside the body cavity, while eye-damaging light can be radiated only within the body cavity.

In a further possible implementation form of the first aspect, each light source arranged in the intracorporeal arm is arranged at a fixed distance from an interior surface of the housing. This ensures correct and identical dosage is applied evenly throughout the body cavity, i.e. at identical distances from the tissue.

In a further possible implementation form of the first aspect, the light sources arranged in the intracorporeal arm are arranged in at least two arrays, the arrays of light sources extending in parallel with the first center axis. This ensures light is radiated evenly across the length and the perimeter of the body cavity.

In a further possible implementation form of the first aspect, the light sources arranged in the extracorporeal arm are arranged partially along the second center axis and partially on opposite sides of the second center axis, facilitating even distribution of light as required.

In a further possible implementation form of the first aspect, the intracorporeal arm and, optionally, the extracorporeal arm has an irregular cross-section, the cross-section extending in a plane perpendicular to the first center axis and the second center axis, respectively. The irregular shape facilitates a shape that is not only comfortable during use but which also is adapted to the shape of the human body.

In a further possible implementation form of the first aspect, the cross-section of the intracorporeal arm has a different shape than the cross-section of the extracorporeal arm, allowing the arms to be adapted to the shapes of different parts of the human body.

In a further possible implementation form of the first aspect, the cross-section is not circular and not elliptical, facilitating a shape that is not only comfortable during use but which is also adapted to the shape of the human body.

In a further possible implementation form of the first aspect, one side of the intracorporeal arm tapers along the direction of the first center axis, such that the distance between the first center axis and the interior surface of the housing is reduced gradually along a first side of the intracorporeal arm while the distance between the first center axis and the interior surface of the housing remains substantially constant along a second side of the intracorporeal arm. This allows the device to be adapted most efficiently to the shape of the human body.

In a further possible implementation form of the first aspect, the device comprises a proximal end and a distal end, the proximal end comprising a free end of the extracorporeal arm and the distal end comprising a free end of the intracorporeal arm, the free end of the intracorporeal arm being configured for insertion into the body cavity.

In a further possible implementation form of the first aspect, the extracorporeal arm has larger external dimensions than the intracorporeal arm, allowing the insertable intracorporeal arm to be more narrow while the extracorporeal arm, which is not intended for insertion, can be made wider and, e.g., accommodate electronics and power supply.

In a further possible implementation form of the first aspect, an area of the housing, where the intracorporeal arm intersects the extracorporeal arm, comprises a recess configured to accommodate the pubic bone of the user, the recess comprising a reduction in cross-section of the intracorporeal arm and of the extracorporeal arm.

In a further possible implementation form of the first aspect, the intracorporeal arm comprises a translucent material configured to allow light in the 380-500 nm spectrum and, optionally, light in the 315-400 nm spectrum, to radiate through the material.

In a further possible implementation form of the first aspect, the extracorporeal arm comprises a first section of translucent material configured to allow light in the 380-500 nm spectrum to radiate through the first section, and a second section of opaque material preventing light in the 380-500 nm spectrum from radiating through the second section.

The foregoing and other objects are achieved by the features of the independent claim. Further implementation forms are apparent from the dependent claims, the description, and the figures. These and other aspects of the invention will be apparent from the embodiments described below.

### BRIEF DESCRIPTION OF THE DRAWINGS

In the following detailed portion of the present disclosure, the invention will be explained in more detail with reference to the example embodiments shown in the drawings, in which:
Fig. 1 is a schematic perspective view of a device in accordance with an example of the embodiments of the disclosure;
Fig. 2 is a schematic side view of a device in accordance with an example of the embodiments of the disclosure;
Fig. 3 is a schematic cross-sectional view of a device in accordance with an example of the embodiments of the disclosure;
Fig. 4 is a schematic top view of a device in accordance with an example of the embodiments of the disclosure.

### DETAILED DESCRIPTION

The present invention relates to a device 1 for emitting light within a body cavity and/or on tissue located outside the body cavity. The body cavity may be a vagina, and the device 1 may be used for vaginal light therapy as well as light therapy of the vulva. Nevertheless, the device may be used for other types of light therapy such as anal light therapy or oral light therapy.

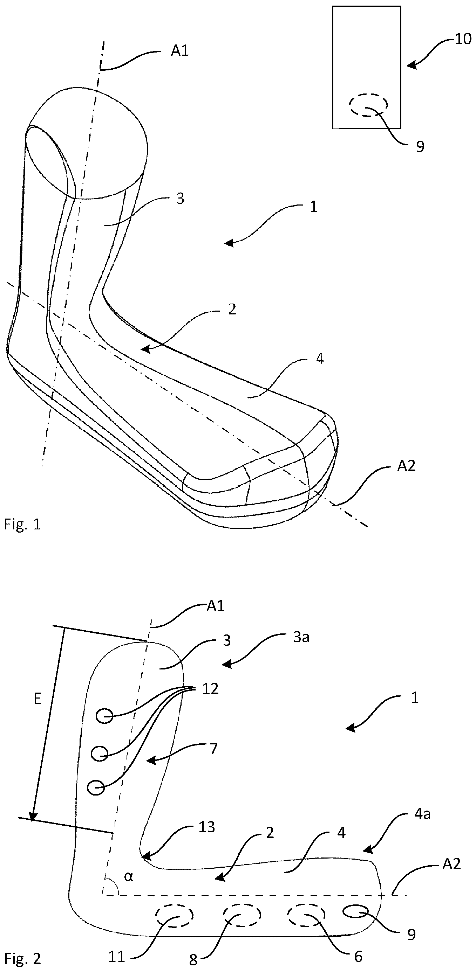

The device comprises a housing 2 which, in turn, comprises an intracorporeal arm 3 having a first center axis A1 and an extracorporeal arm 4 having a second center axis A2. The first center axis A1 intersects the second center axis A2, as illustrated in Figs. 1 to 3. The first center axis A1 and the second center axis A2 can be considered the longitudinal axis of the respective arm. As shown in Figs. 1 and 2, the first center axis A1 may extend at an angle α to the second center axis A2, the angle α being 45°<α≤90°, preferably 75°≤α≤90°.

The intracorporeal arm 3 is configured to be inserted into a body cavity while the extracorporeal arm 4 is configured to extend outside of the body cavity when the intracorporeal arm 3 is inserted into the body cavity. The intracorporeal arm 3 and the extracorporeal arm 4 can both be considered to be legs or elongated tubular members with varying cross-sections.

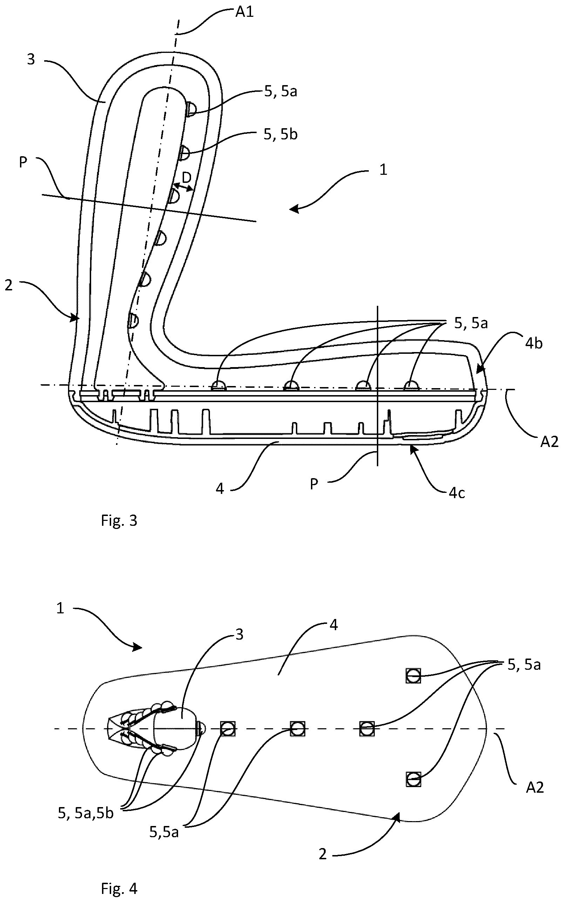

The housing 2 may be substantially L-shaped as shown in Fig. 4 when the angle α is one of the larger values included in the above-mentioned range, substantially V-shaped when the angle α is one of the smaller values included in the above-mentioned range (not shown), or substantially T-shaped when the intracorporeal arm 3 is connected to a middle portion of the extracorporeal arm 4 (not shown). The L-, V- or T-shape is, in other words, formed by the intracorporeal arm 3 and the extracorporeal arm 4. The device 1 may comprise a proximal end and a distal end, the proximal end comprising a free end 4a of the extracorporeal arm 4 and the distal end comprising a free end 3a of the intracorporeal arm 3, the free end 3a of the intracorporeal arm being configured for insertion into the body cavity.

The housing 2 comprises a translucent material configured to allow propagation of light through the housing 2. The translucent material may be UV-resistant. The translucent material may be frosted or etched to improve light distribution.

The intracorporeal arm 3 may comprise a translucent material configured to allow light in the 380-500 nm spectrum and, optionally, light in the 315-400 nm spectrum, to radiate through the material. As illustrated in Fig. 3, the extracorporeal arm 4 may comprise a first section 4b of translucent material configured to allow light in the 380-500 nm spectrum to radiate through the first section, and a second section 4c of opaque material preventing light in the 380-500 nm spectrum from radiating through the second section. The first section 4b of translucent material may be configured to abut an exterior surface of the human body such as the vulva. The second section 4c of opaque material may be configured to be directed away from an exterior surface of the human body such as the vulva, i.e. the second section 4c of opaque material may form an impenetrable barrier preventing light from propagating in directions away from the body. The opaque material may be completely opaque silicone hiding components arranged within the extracorporeal arm 4 and providing an improved grip to help insertion.

The intracorporeal arm 3 and at least a part of the extracorporeal arm 4 may be a single-piece unit, such as an injection molded piece. For example, the second section 4c of the extracorporeal arm 4 may be a separate component while the remainder of the extracorporeal arm 4 and the intracorporeal arm 3, i.e. the remainder of the housing 2, is a single-piece unit. In such an embodiment, the second section 4c may be detachably connected to the first section 4b, e.g. by means of snap-fit.

An energy source 6 is arranged within the housing 2. The energy source 6 may be a battery and the battery may be arranged within the extracorporeal arm 4. The battery 6 may be a rechargeable battery and the device 1 may be configured to be charged by means of, for example, an inductive charger capable of cradling the device 1.

A plurality of light sources 5 are arranged within the housing 2. The plurality of light sources 5 may be arranged in the intracorporeal arm 3 and/or in the extracorporeal arm 4 and are used to treat tissue with light of specific wavelengths. If only the light sources 5, 5a, 5b arranged in the intracorporeal arm 3 are activated, only tissue within the body cavity is treated. If only the light sources 5, 5a arranged in the extracorporeal arm 4 are activated, only tissue outside, but in direct connection with, the body cavity is treated. Activation may be adapted such that only light sources 5, 5a, 5b arranged in the intracorporeal arm 3 are activated, only light sources 5, 5a arranged in the extracorporeal arm 4 are activated, or all light sources 5, 5a, 5b arranged in the intracorporeal arm 3 as well as the extracorporeal arm 4 are activated.

The plurality of light sources 5 may comprise light emitting diodes configured to emit light in a 380-500 nm spectrum and, optionally, in a 315-400 nm spectrum. Light sources 5, 5a configured to emit light in the 380-500 nm spectrum may be arranged in the intracorporeal arm 3 and in the extracorporeal arm 4, while light sources 5b configured to emit light in the 315-400 nm spectrum may be arranged in the intracorporeal arm 3 only.

As illustrated in Fig. 3, light sources 5 arranged in the intracorporeal arm 3 may be arranged at a fixed distance D from an interior surface of the housing 2. The light sources 5 may, for example, be arranged such that the distance D is 5 mm. With a housing thickness of 3 mm, this would make the distance between light source and tissue 8 mm.

Light sources 5 arranged in the intracorporeal arm 3 may be arranged in at least two arrays extending in parallel with the first center axis A1. As illustrated in Fig. 4, the light sources 5 may be arranged in three arrays, the arrays being substantially equidistantly arranged along the circumference of the intracorporeal arm 3. The light sources 5a, 5b within each array may be arranged alternatingly such that adjacent light sources 5 have different wavelengths.

As also illustrated in Fig. 4, the light sources 5 arranged in the extracorporeal arm 4 may be arranged partially along the second center axis A2, for example where the extracorporeal arm 4 is more narrow, and partially on opposite sides of the second center axis A2, for example where the extracorporeal arm 4 is wider. For example, the extracorporeal arm 4 may be 1.5-2 times wider than the intracorporeal arm 3 at its widest.

A sensor arrangement 7 is configured to detect if the intracorporeal arm 3 is inserted, to a predefined extent E as illustrated in Fig. 1, within the body cavity. The intracorporeal arm 3 may be considered to be "located to the predefined extent E within the body cavity" when all light sources 5 of the intracorporeal arm 3 are located within the body cavity. In other words, the sensor arrangement 7 may be configured to detect if the intracorporeal arm 3 is located, to the predefined extent E, within the body cavity by detecting that the intracorporeal arm 3 has been inserted into an orifice enveloping the intracorporeal arm 3.

A microprocessor 8 is configured to allow activation of at least one of the light sources 5 and to allow continuous emission of light from the light sources 5 as long as the sensor arrangement detects that the intracorporeal arm is inserted, to the predefined extent E, within the body cavity.

A user interface 9 is connected to the microprocessor 8 for activating the light sources 5. The user interface 9 may be an analogue interface arranged on the extracorporeal arm 4 and/or a digital interface downloaded on a digital apparatus 10 connected to the device 1. The analogue interface may be a button and the digital interface may be an application, as illustrated in Fig. 1. The user interface 9 may allow activation of the light sources 5 and/or pausing or deactivation of the light sources 5.

Each light source 5 may be deactivated by means of the user interface 9, by means of a timer connected to the microprocessor 8, e.g. deactivating the light sources 5 automatically after 30 minutes, and/or by means of the sensor arrangement 7 detecting extraction of the intracorporeal arm 3 from the body cavity.

The device 1 may further comprise wireless technology 11 allowing transmission of data from the microprocessor 8 to the user interface 9 and, optionally, from the user interface 9 to the microprocessor 8. Transmission of data from the microprocessor 8 to the user interface 9, for example, allows a user to monitor progress during use. Transmission of data from the user interface 9 to the microprocessor 8 may be utilized to control the operation of the device. The wireless technology 11 may be configured to operate under the industrial, scientific, and medical (ISM) frequency range (2400-2500MHz), via protocols such as Bluetooth, Wi-Fi, Zigbee, etc.

The sensor arrangement 7 may comprise a plurality of sensors 12 arranged in the intracorporeal arm 3, as illustrated in Fig. 2. The sensor arrangement 7 may be configured to sequentially detect the insertion of the intracorporeal arm 3 into the body cavity and/or extraction of the intracorporeal arm 3 from the body cavity. The sequential detection may be achieved by means of at least one array of sensors 12 arranged in the intracorporeal arm 3, the array extending substantially parallel with the first center axis A1. Each sensor 12 detects, in sequence, whether the section comprising the specific sensor is inserted into a body cavity. When all sensors 12 in an array have detected insertion into the body cavity, it is determined, by means of the microprocessor 8, that the intracorporeal arm 3 has been sufficiently inserted into the body cavity and the light sources 5 may be activated.

The sequential detection ensures that erroneous determination of the intracorporeal arm 3 being sufficiently inserted into the body cavity is avoided, for example when the intracorporeal arm 3 and associated sensors 12 are grasped by hand. The corresponding applies to deactivation of the light sources 5 upon extraction of the intracorporeal arm 3 from the body cavity, as the sensors 12 of at least one array detect, in sequence, that the intracorporeal arm 3 is no longer sufficiently inserted into the body cavity. As a consequence, the light sources 5 are deactivated immediately.

The sensor arrangement may comprise at least one of a capacitive sensor, a light sensor, and/or a temperature sensor. The temperature sensor may be configured to register the body temperature of the user.

The intracorporeal arm 3 and, optionally, the extracorporeal arm 4 may have an irregular cross-section, as best illustrated in Fig. 4. The cross-section extends in a plane P perpendicular to the first center axis A1 and the second center axis A2, respectively. Furthermore, the cross-section of the intracorporeal arm 3 may have a different shape than the cross-section of the extracorporeal arm 4. The cross-section is not circular and not elliptical. The extracorporeal arm 4 may have larger external dimensions than the intracorporeal arm 3.

One side of the intracorporeal arm 3 may taper along the direction of the first center axis A1, such that the distance between the first center axis A1 and the interior surface of the housing 2 is reduced gradually along a first side of the intracorporeal arm 3, while the distance between the first center axis A1 and the interior surface of the housing 2 remains substantially constant along a second side of the intracorporeal arm. Fig. 3 shows the first side of the intracorporeal arm 3 to the right and the second side of the intracorporeal arm 3 to the left of first center axis A1.

An area of the housing 2, where the intracorporeal arm 3 intersects or connects to the extracorporeal arm 4, may comprise a recess 13 configured to accommodate a pubic bone of a user, the recess 13 comprising a reduction in cross-section of the intracorporeal arm 3 and of the cross-section of the extracorporeal arm 4. In other words, the area of the device 1 where the intracorporeal arm 3 and the extracorporeal arm 4 interconnect may comprise a recess shaped to provide space for the pubic bone of the user.

The invention has been described in conjunction with various embodiments herein. However, other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

## Claims

1. A device (1) for emitting light within a body cavity and/or on tissue outside said body cavity, the device comprising:
- a housing (2) comprising an intracorporeal arm (3) having a first center axis (A1) and an extracorporeal arm (4) having a second center axis (A2), said first center axis (A1) intersecting said second center axis (A2),
said housing (2) comprising a translucent material configured to allow propagation of light through said housing (2);
- a plurality of light sources (5) arranged within said housing (2);
- an energy source (6) arranged within said housing (2);
- a sensor arrangement (7) configured to detect if said intracorporeal arm (3) is inserted, to a predefined extent (X), within said body cavity;
- a microprocessor (8) configured to allow activation of at least one of said light sources (5) and to allow continuous emission of light from said light source(s) (5) as long as said sensor arrangement detects that said intracorporeal arm is inserted, to said predefined extent (X), within said body cavity;
- a user interface (9) connected to said microprocessor (8) for activating said light source(s) (5).

2. The device (1) according to claim 1, wherein said first center axis (A1) extends at an angle (α) to said second center axis (A2), said angle (α) being 45°<α≤90°, preferably 75°≤α≤90°.

3. The device (1) according to any one of the previous claims, wherein each light source (5) is deactivated by means of said user interface (9), by means of a timer connected to said microprocessor (8), and/or by means of said sensor arrangement (7) detecting extraction of said intracorporeal arm (3) from said body cavity.

4. The device (1) according to any one of the previous claims, wherein said user interface (9) is an analogue interface arranged on said extracorporeal arm (4) and/or a digital interface downloaded on a digital apparatus (10) connected to said device (1).

5. The device (1) according to any one of the previous claims, further comprising wireless technology (11) allowing transmission of data from said microprocessor (8) to said user interface (9) and, optionally, from said user interface (9) to said microprocessor (8).

6. The device (1) according to any one of the previous claims, wherein said sensor arrangement (7) comprises a plurality of sensors (12) arranged in said intracorporeal arm (3).

7. The device (1) according to claim 6, wherein said sensor arrangement (7) is configured to sequentially detect insertion of said intracorporeal arm (3) into said body cavity and/or extraction of said intracorporeal arm (3) from said body cavity, said sequential detection being achieved by means of at least one array of sensors (12) arranged in said intracorporeal arm (3), said array extending parallel with said first center axis (A1).

8. The device (1) according to any one of the previous claims, wherein said sensor arrangement comprises at least one of a capacitive sensor, a light sensor, and/or a temperature sensor.

9. The device (1) according to any one of the previous claims, wherein said plurality of light sources (5) are arranged in said intracorporeal arm (3) and/or in said extracorporeal arm (4).

10. The device (1) according to any one of the previous claims, wherein said plurality of light sources (5) comprises light emitting diodes configured to emit light in a 380-500 nm spectrum and, optionally, in a 315-400 nm spectrum.

11. The device (1) according to claim 10, wherein light sources (5) configured to emit light in said 380-500 nm spectrum are arranged in said intracorporeal arm (3) and in said extracorporeal arm (4), and wherein light sources (5) configured to emit light in said 315-400 nm spectrum are arranged in said intracorporeal arm (3).

12. The device (1) according to any one of the previous claims, wherein each light source (5) arranged in said intracorporeal arm (3) is arranged at a fixed distance (D) from an interior surface of said housing (D).

13. The device (1) according to any one of the previous claims, wherein said intracorporeal arm (3) and, optionally, said extracorporeal arm (4) has an irregular cross-section, said cross-section extending in a plane (P) perpendicular to said first center axis (A1) and said second center axis (A2), respectively.

14. The device (1) according to any one of the previous claims, wherein an area of said housing (2), where said intracorporeal arm (3) intersects said extracorporeal arm (4), comprises a recess (13) configured to accommodate a pubic bone of a user, said recess (13) comprising a reduction in cross-section of said intracorporeal arm (3) and of said extracorporeal arm (4).

15. The device (1) according to any one of the previous claims, wherein said intracorporeal arm (3) comprises a translucent material configured to allow light in said 380-500 nm spectrum and, optionally, light in said 315-400 nm spectrum, to radiate through said material.

16. The device (1) according to any one of the previous claims, wherein said extracorporeal arm (4) comprises a first section (4b) of translucent material configured to allow light in said 380-500 nm spectrum to radiate through said first section, and a second section (4c) of opaque material preventing light in said 380-500 nm spectrum from radiating through said second section.
